# FASCICULE DE BREVET EUROPEEN

(11) **EP 1 952 783 B1**
(45) Date de publication et mention de la délivrance du brevet: **20.04.2011**
(21) Numéro de dépôt: 08150746.9
(22) Date de dépôt: 29.01.2008
(51) Int. Cl.: A61C 19/045

(54) **Dispositif de reperage et de mesure de parametres anatomiques faciaux**
Vorrichtung zur Markierung und Messung von anatomischen Parametern des Gesichts
Device for marking and measuring anatomical facial parameters

(30) Priorité: 02.02.2007 FR 0753049
(43) Date de publication de la demande: 06.08.2008
(73) Titulaire: Margossian, Patrice, 13008 Marseille (FR)
(72) Inventeur: Margossian, Patrice, 13008 Marseille (FR)
(74) Mandataire: Domange, Maxime

(56) Documents cités:
- EP-A1- 1 600 117
- DE-C1- 4 025 684
- US-A- 4 547 154
- US-A- 4 634 377
- US-A- 6 109 917
- US-A1- 2005 277 086
- US-A1- 2006 003 285
- US-A1- 2006 172 254
- US-B1- 6 386 868

## Description

La présente invention concerne un dispositif de repérage et de mesure permettant de mesurer de façon précise les paramètres structurels du visage d'un sujet, de façon à en évaluer l'harmonie esthétique ou, le cas échéant, à déterminer de manière quantifiable les modifications nécessaires pour obtenir une telle harmonie par un biais chirurgical et/ou prothétique.

Les domaines d'application du dispositif de l'invention sont nombreux et incluent notamment dans l'odontologie, la stomatologie, l'orthodontie et la chirurgie plastique et réparatrice du visage humain.

Le dispositif de l'invention est plus particulièrement conçu pour faciliter les opérations de reconstruction et de réparation dentaires et parodontales dans le respect de l'harmonie esthétique du visage d'un sujet, ainsi que les opérations de chirurgie esthétique de la sphère buccale, pour le rétablissement d'une harmonie esthétique du visage.

On considère traditionnellement, sur un plan médical, qu'un visage présente une esthétique harmonieuse lorsqu'un ensemble de critères, notamment de parallélisme, de perpendicularité et de symétrie, sont respectés.

Ces critères concernent particulièrement cinq lignes particulières du visage qui sont:
➢ la ligne bi-pupillaire, c'est-à-dire la ligne passant par les deux pupilles des yeux du sujet,
➢ la ligne ophriatique, c'est-à-dire la ligne passant par les bords supérieurs des deux sourcils du sujet,
➢ la ligne bi-commissurale, c'est-à-dire la ligne passant par les commissures des lèvres du sujet,
➢ la ligne des bords libres des dents du maxillaire antérieur ou ligne incisive ou encore, par abus de langage, « ligne du sourire » et
➢ la ligne des contours gingivaux maxillaires antérieurs.

Ces cinq lignes sont complétées avec le plan de Camper, plan défini par les deux lignes joignant les points sous-nasaux et les porions de chaque côté du visage ou, de manière similaire, défini par l'horizontale et la ligne passant par le point sous nasal et les porions (ou les tragus). Le plan d'occlusion idéal s'inscrit dans un plan parallèle à ce plan de Camper.

Ces cinq lignes doivent en outre être perpendiculaires au plan sagittal médian de la face du sujet dans lequel s'inscrivent idéalement l'arête du nez, le philtrum et la ligne inter-incisive.

Bien que ces règles soient admises collectivement, les mesures sont à ce jour effectuées de manière approximative et subjective par les praticiens concernés, qui se basent soit sur leur ressenti visuel soit sur des procédés aléatoires de mesure à main levée, faute d'outil de matérialisation et mesure objectif des différents critères esthétiques définis ci-avant.

Or, le repérage précis de ces paramètres esthétiques est essentiel pour déterminer et quantifier les dissymétries du visage et/ou de la mâchoire de chaque patient, mesurer ces dissymétries, et déterminer en conséquence les profils, dimensionnement et orientation des opérations de reconstruction à opérer pour rétablir l'harmonie esthétique du visage du patient, et notamment la réalisation de prothèses dentaires maxillaires et/ou mandibulaires ainsi que leur intégration parodontale.

En matière de reconstruction dentaire, la vérification et le respect du parallélisme de la ligne incisive idéale avec la ligne bi-pupillaire et de la perpendicularité de cette dite ligne incisive idéale avec le plan sagittal médian, de même que la vérification de la symétrie de l'implantation dentaire des patients par rapport à ce plan sagittal médian sont essentiels à l'obtention d'une esthétique harmonieuse.

La difficulté principale à ce jour pour les praticiens dans le cadre de traitements de restauration dentaire et parodontale réside dans le respect simultané des critères fonctionnels d'occlusion et des critères d'harmonie esthétique du visage.

Le respect de ces critères esthétiques ne peut cependant pas découler directement du respect des critères anatomiques fonctionnels d'occlusion des patients traités, critères fonctionnels qui sont aujourd'hui maîtrisés et déterminables par les praticiens grâce à des outils tels que les arcs faciaux et les articulateurs.

Or les arcs faciaux permettent l'enregistrement de la position spatiale du maxillaire supérieur du patient par rapport à un plan de référence qui est le plan de Frankfort et ensuite son transfert sur les articulateurs pour la réalisation de prothèses maxillaires complètes ou partielles et l'ajustement de l'occlusion dentaire en harmonie avec la physiologie articulaire du patient avant implantation sur les patients.

Ces arcs faciaux, dont les documents US 2006/0003285 A1EP 1 600 117 A, US 6 109 917 et US 7 048 539 B2 donnent divers exemples, ne permettent pas un repérage des paramètres esthétiques du visage. Ils ne permettent pas non plus de diagnostiquer les dissymétries dentaires par rapport à ces paramètres esthétiques.

De plus, les arcs faciaux s'appuyant sur des parties molles et mobiles du visage ne permettent pas un enregistrement fiable et reproductible de la position maxillaire. En outre il est très fréquent d'observer une asymétrie entre les plans et axes de référence fonctionnels d'une part et la ligne bi-pupillaire qui est la référence esthétique d'autre part, faussant inévitablement le repérage des paramètres esthétiques.

On ne connaît pas à ce jour d'outil fiable pour réaliser de manière précise et reproductible le repérage et la mesure des paramètres esthétiques du visage. Ces mesures sont effectuées de manière approximative et subjective par les praticiens, qui se basent soit sur leur ressenti visuel, soit sur des procédés aléatoires de mesure à main levée, faute d'outil de mesure objectif.

Le but de l'invention est de procurer un dispositif permettant de visualiser et repérer sur le visage de patients les paramètres d'évaluation de l'harmonie esthétique du visage de manière simultanée, fiable et reproductible.

Les positionnements de ces lignes sont variables d'un individu à un autre et le dispositif selon l'invention permet précisément de repérer les lignes les unes par rapport aux autres pour chaque individu particulier. Ces repères servent ensuite pour la création d'une prothèse à la fois fonctionnelle et esthétique. Le but du repérage d'au moins une partie de ces lignes est donc de créer un plan de reconstruction prothétique qui respecte les particularités du visage de l'individu tout en lui assurant des critères esthétiques reconnus.

Un autre but de l'invention est donc de fournir un dispositif qui permette de transférer les paramètres esthétiques repérés sur le visage d'un patient sur les moulages en plâtre de ses propres arcades dentaires qui, une fois réalisés, seront utilisés sur les articulateurs pour la fabrication de prothèses dentaires, assurant ainsi à la fois fonctionnalité physiologique, articulaire et occlusale des prothèses et harmonie esthétique optimale pour le patient après mise en place.

L'invention atteint ses buts grâce à un dispositif de repérage et de mesure de paramètres anatomiques faciaux du visage d'un patient, selon la revendication 1.

Le dispositif selon l'invention est particulièrement avantageux dans la mesure où il permet de fournir au praticien un outil d'évaluation simple et précis de l'esthétique du visage d'un patient, de ses éventuels défauts et de la mesure des corrections prothétiques à apporter dans le cadre d'applications de réparation ou de reconstruction dentaire ou parodontale.

Selon l'invention, lesdits moyens de matérialisation et de positionnement de la ligne incisive, de la ligne bi-pupillaire et les moyens d'alignement du plan de Camper sont respectivement solidaires dudit cadre. De la sorte, tous les paramètres esthétiques à repérer individuellement d'une part et en relation avec les autres paramètres d'autre part sont liés à un même support fixe ce qui améliore la justesse du repérage.

Lesdits moyens de matérialisation et de positionnement de la ligne incisive et de la ligne bi-pupillaire étant horizontaux, aptes à coulisser perpendiculairement sur ledit cadre et de préférence montés sur au moins un des montants dudit cadre, il est ainsi très simple et rapide de procéder au positionnement et au repérage de ces deux repères esthétiques sur le visage d'un patient.

Grâce à l'invention, le praticien peut positionner précisément, visualiser et fixer, sur le cadre du dispositif de l'invention, la ligne bi-pupillaire, la ligne incisive et l'orientation du plan de Camper d'un patient. Il peut ensuite ainsi utiliser ces repères pour les utiliser sur les moulages dentaires de plâtre servant à la réalisation des prothèses pour le patient afin d'assurer le respect des paramètres esthétiques du visage après la mise en place des prothèses.

Grâce à l'invention, le praticien peut aussi faire du diagnostic en bouche en observant les éventuelles dissymétries ou défauts de parallélisme entre certains éléments anatomiques. Par exemple, le praticien pourra repérer avec exactitude un défaut de parallélisme de la ligne incisive avec la ligne bi-pupillaire ou encore un défaut de parallélisme du plan d'occlusion avec le plan de Camper. Les mesures et observations faites avec le dispositif selon l'invention peuvent donc ensuite être utilisées pour évaluer les besoins du patient et le travail à effectuer.

La fixation de la fourchette de morsure offre, selon l'invention, une excellente mobilité du cadre par rapport au visage du patient qui la mord et cela facilite le réglage et le positionnement du dispositif de l'invention sur le visage du patient.

Avantageusement, les moyens de matérialisation et de positionnement de la ligne incisive, et les moyens de matérialisation et de positionnement de la ligne bi-pupillaire comportent des moyens de blocage sur les montants dudit cadre dans une position de repérage souhaitée.

Avec une telle caractéristique, on assure que les positionnements des différentes lignes et plans observés sur le patient et reportés sur le dispositif selon l'invention ne bougent pas, par exemple lors du transport du dispositif selon l'invention entre l'officine du dentiste et celle du prothésiste.

Selon une autre caractéristique avantageuse de l'invention, ladite fourchette de morsure peut notamment être fixée sur la traverse inférieure dudit cadre par l'intermédiaire d'une pièce de liaison articulée comprenant des moyens de serrage variable permettant au moins des déplacements et blocages de ladite fourchette en translation et en rotation sur ladite traverse et autour de l'axe de sa tige de fixation.

Selon une caractéristique préférentielle, ladite fourchette de morsure comprend des moyens d'enregistrement capable de garder trace de la position des surfaces dentaires du patient lorsqu'il mord la fourchette.

Cette caractéristique assure, lorsque le dispositif est ultérieurement utilisé pour trace des éléments de reconstruction sur un modèle en plâtre, une position correcte du modèle en plâtre moulé par rapport au dispositif, cette position correcte étant celle qu'a le patient lors de la prise de mesure.

Conformément à l'invention, ladite fourchette de morsure comporte également des patins d'écartement des mâchoires lorsque ladite fourchette est mordue par un dit patient, de manière à provoquer chez le patient une légère désocclusion permettant, par contraste avec l'intérieur de la bouche de repérer exactement la ligne incisive au niveau du bord incisif du maxillaire supérieur. De plus, la béance évite également au praticien pratiquant les mesures d'être gêné par la proximité des lèvres.

Conformément à une autre caractéristique préférée de l'invention, les moyens d'alignement du plan de Camper forment un angle α compris entre 60° et 80° par rapport à au moins un des montants dudit cadre. Un tel angle d'orientation correspond avantageusement aux valeurs moyennes extrêmes d'orientation du plan de Camper par rapport à un plan perpendiculaire au plan esthétique. Ce plan esthétique se situe en moyenne 8° au dessus du plan de Frankfort et donne le plan horizontal de la face lorsque le patient regarde au loin. Le plan de Camper (porion/point sous nasal) se situe quant à lui environ 20 ° en dessous du plan de Frankfort (porion/point infra orbitaire); il est également par convention parallèle au plan d'occlusion du patient.

De façon simple et avantageuse, les moyens d'alignement du plan de Camper comportent au moins une tige rectiligne fixée sur au moins un des montants dudit cadre, et qui s'étend dans un plan perpendiculaire aux dits moyens de matérialisation et de positionnement de la ligne incisive et de la ligne bi-pupillaire.

Cette caractéristique est avantageuse mais la perpendicularité de la ou des tiges avec les moyens de matérialisation et de positionnement de la ligne incisive et de la ligne bi-pupillaire n'est pas nécessaire, la tige pouvant appartenir à un plan passant par le montant du cadre et faisant un angle non droit avec le plan du cadre.

Aussi on note que selon une caractéristique préférentielle de l'invention, lors de l'utilisation du dispositif, une tige rectiligne est fixée, à des hauteurs identiques, sur chacun des deux montants. On verra que cette caractéristique permet le placement facilité d'un outil de transfert qui sera porté par les deux tiges qui se font face de part et d'autre du cadre. On assurera alors la perpendicularité de l'outil avec les montants du cadre.

Dans un mode de réalisation préféré du dispositif de l'invention, lesdits moyens de matérialisation et de positionnement de la ligne incisive et de la ligne bi-pupillaire comportent respectivement un fil fixé de façon coulissante aux montants du cadre et un réglet transparent monté dans au moins une glissière sur au moins un des montants du cadre.

Des moyens de blocage dudit fil et dudit réglet sur les montants dudit cadre dans une position de repérage souhaitée seront avantageusement implémentés pour bloquer ce fil ou ce réglet en position.

Dans un mode de réalisation particulier, le dispositif de l'invention comporte également un moyen de positionnement de l'axe sagittal médian du visage. De préférence, ce dernier se trouve solidaire des moyens de matérialisation et de positionnement de la ligne bi-pupillaire ce qui permet de repérer simultanément et précisément la ligne bi-pupillaire et l'axe sagittal médian du visage.

Pour améliorer encore ce repérage, lesdits moyens de matérialisation et de positionnement de la ligne bi-pupillaire comportent également des graduations permettant le centrage dudit moyen de positionnement de l'axe sagittal médian par rapport aux pupilles dudit patient.

Enfin, dans une ultime variante de réalisation, le dispositif peut également comporter des moyens de matérialisation et de positionnement de la ligne incisive du maxillaire inférieur afin de permettre un rétablissement du parallélisme des dents du maxillaire inférieur avec celles du maxillaire supérieur et la ligne bi-pupillaire.

De façon préférée, ces moyens de positionnement de la ligne incisive inférieure sont identiques à ceux de la ligne incisive du maxillaire supérieur, c'est-à-dire qu'ils comportent un fil fixé sur les montants du cadre du dispositif et des graduations de réglage de la position dudit fil.

Une autre caractéristique avantageuse du dispositif de l'invention réside dans la fourniture d'un outil de transfert de la ligne incisive idéale et de l'axe sagittal médian sur un moulage de l'arcade maxillaire en plâtre des patients traités, cet outil de transfert étant adapté pour venir se placer sur les moyens d'alignement du plan de Camper.

On remarque que, selon l'invention, l'outil de transfert étant positionné sur les moyens de positionnement du plan de Camper, il est tel qu'il permet également le transfert d'une ligne tridimensionnelle sur l'avant et les cotés du modèle, cette ligne appartenant à un plan parallèle au plan de Camper et étant, sur l'avant du modèle, parallèle à la ligne bi-pupillaire.

Cet outil de transfert peut revêtir une forme très simple, comme par exemple étant constitué d'un plateau apte à être placé ou glissé sur les tiges d'alignement du plan de Camper, en butée contre les montants du cadre, et comportant une échancrure ou ouverture postérieure permettant l'ajustement du plateau contre un moulage en plâtre du maxillaire placé sur la fourchette du dispositif. Le plateau est en outre muni de découpes frontales permettant le placement en butée dudit plateau contre les montants du cadre et, perpendiculairement aux montants du cadre, en appui sur lesdits moyens d'alignement du plan de Camper.

Le plateau vient alors se positionner autour du moulage. Par constitution même du dispositif selon l'invention, le plateau définit alors un plan parallèle au plan de Camper et à la ligne bi-pupillaire. Ainsi, il est ensuite très aisé, à l'aide d'un crayon par exemple, d'utiliser le plateau comme un guide pour établir sur le moulage en plâtre une ligne parallèle à la ligne bi-pupillaire et également parallèle au plan de Camper. En effet, la ligne est alors parallèle à la ligne bi-pupillaire sur l'avant du moulage en plâtre et parallèle au plan de Camper sur les cotés du moulage en plâtre.

Avantageusement, un tel plateau de transfert peut également comporter une languette, pivotante ou fixe, situé dans le plan vertical médian de l'échancrure postérieure et donc virtuellement dans l'axe sagittal médian lorsque le plateau est positionné sur les tiges latérales du dispositif, cette languette comportant une rainure ou une ouverture centrale permettant le tracé sur le modèle maxillaire de plâtre d'un patient de la ligne sagittale médiane, telle que repérée précédemment à l'aide du dispositif sur le visage du patient.

Selon l'invention, le plateau de transfert peut aussi porter un dispositif d'émission lumineuse apte à projeter une ligne, pour tracer l'axe sagittal médian, ou une croix, pour tracer l'axe sagittal médian en même temps que la parallèle à la ligne bi-pupillaire et au plan de Camper, sur le moulage en plâtre alors recouvert d'un vernis photosensible. Le changement d'aspect du vernis engendré sous l'effet de la lumière émise permet de reporter directement l'axe sagittal médian sur le moulage. Le dispositif d'émission lumineuse est préférentiellement un dispositif laser. Eventuellement, le dispositif d'émission peut être mobile en translation sur le plateau afin notamment de permettre de choisir la taille de la figure géométrique tracée.

Avantageusement, la languette, dispositif de guidage, est munie de rainures traversantes ou ouvertures en T ou en croix permettant le tracé sur un dit modèle maxillaire de plâtre d'une ligne représentative de l'axe sagittal médian du visage du patient et d'une ligne parallèle à la ligne bi-pupillaire.

Les caractéristiques du dispositif de l'invention, les avantages et l'utilisation particulière de celui-ci ressortiront mieux à la lecture de la description détaillée qui va suivre, effectuée en référence aux figures annexées illustrant le dispositif de l'invention dans un mode particulier de réalisation et parmi lesquelles:
- la figure 1 représente une vue en perspective du dispositif de repérage et de mesure de l'invention;
- les figures 2A et 2B représentent en perspective et en vue de dessus une fourchette de morsure adaptée au dispositif de l'invention;
- la figure 3 représente en vue de face le mode d'utilisation et de repérage de paramètres esthétiques sur le visage d'un patient à l'aide du dispositif de l'invention;
- la figure 4 représente en vue de profil le mode d'utilisation et de repérage de paramètres esthétiques sur le visage d'un patient à l'aide du dispositif de l'invention;
- les figures 5A à 5C représentent respectivement, en perspective et en vue de gauche, un premier mode de réalisation d'un plateau de transfert de repères du dispositif de l'invention et un second mode de réalisation d'un plateau de transfert de repères du dispositif de l'invention ;
- les figures 6A et 6B représentent en vue de profil et en vue de face le mode de transfert de lignes de reconstructions esthétiques sur un modèle maxillaire en plâtre à l'aide du dispositif de l'invention et à partir des données esthétiques relevées sur un patient.

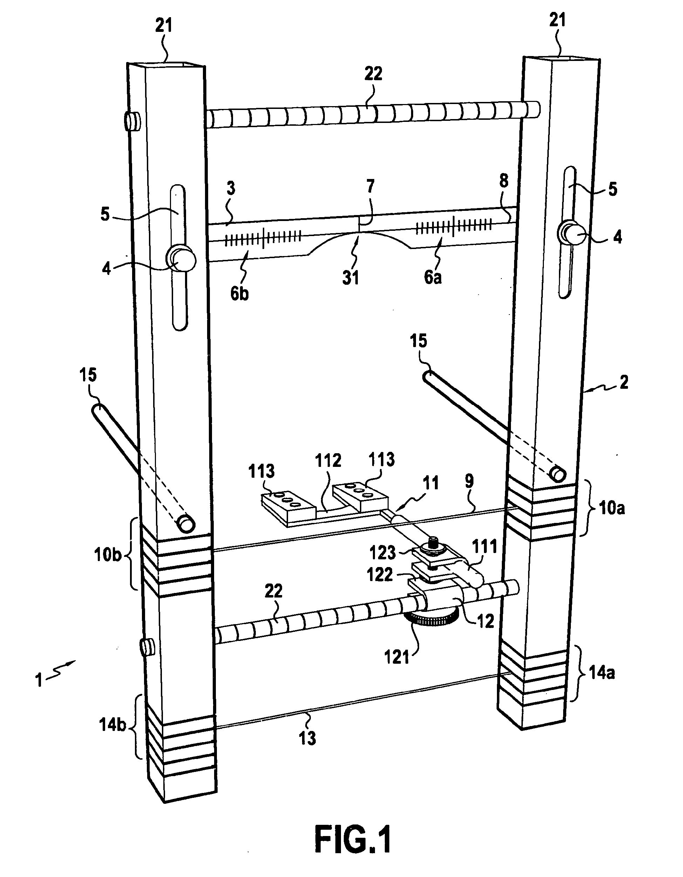

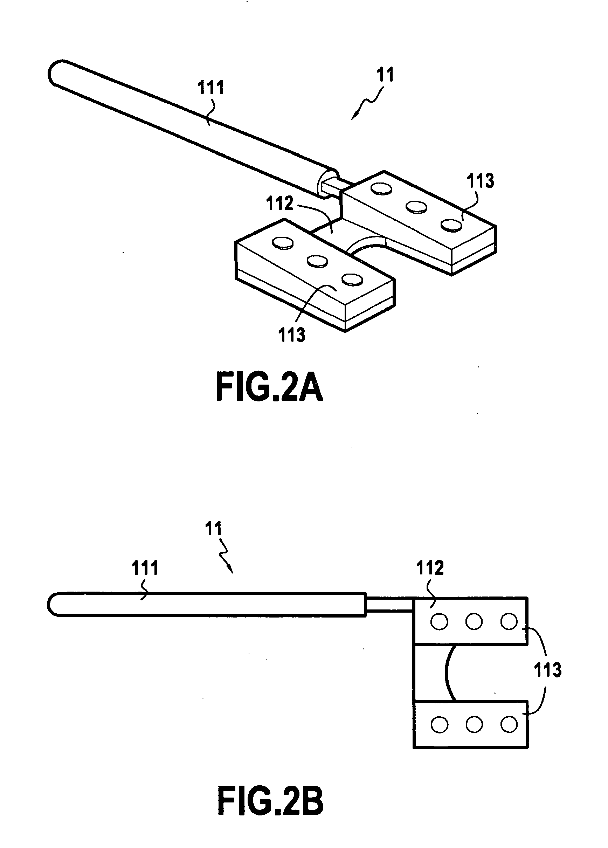

En référence tout d'abord aux figures 1, 2A et 2B, le dispositif de repérage et de mesure 1 de la présente invention comporte un cadre 2 composé de deux montants verticaux 21 reliés entre eux au niveau de leurs extrémités supérieures et inférieures respectives par deux traverses 22. Dans l'exemple de réalisation représenté, les montants 21 sont de section sensiblement carrée ou rectangulaire et les traverses 22 de section circulaire. Cependant cette forme particulière de montants et de traverses ne revêt absolument aucun caractère limitatif et d'autres formes peuvent être utilisées sans grever les fonctionnalités du dispositif 1.

Les dimensions du cadre 2 sont choisies de telle sorte que tous les types de visage humain puissent s'inscrire dans la surface interne du cadre lorsque le dispositif est mis en place pour la prise de repères.

Les montants 21 portent en partie haute une latte 3, également appelée réglet, de Polyméthacrylate de Méthyle (PMMA), plus connu sous le nom commercial de Plexiglas®, fixée aux montants 21 par l'intermédiaire de moyens de réglage et de blocage 4. Lesdits moyens de réglage 4 comportent un boulon 41 dont la vis est insérée dans une rainure débouchante 5 formée dans l'épaisseur de chacun des montants 21.

A une première extrémité, la vis des boulons 41 porte un bouton de manipulation. L'extrémité opposée de la vis des boulons 41 est, elle, libre et traverse un orifice pratiqué aux extrémités de ladite latte de PMMA 3 de manière à porter celle-ci et permettre son réglage en hauteur par coulissement dans les rainures 5 des montants. Le serrage et blocage de la latte 3 est assuré par un écrou vissé à l'extrémité libre de la vis des boulons 41 et plaquant la latte 3 contre la face arrière des montants 22. La latte 3 est telle qu'elle peut être mobilisée tout en restant perpendiculaire aux montants 21. En tout cas, lors de l'utilisation du dispositif, le dispositif est tel qu'il assure que la latte 3 est perpendiculaire aux montants 21 lorsque cette latte 3 est alignée avec la ligne bi-pupillaire.

La latte 3 de PMMA comporte deux séries de graduations 6a, 6b disposées de façon symétrique par rapport à une ligne verticale médiane 7 tracée sur la latte 3, elle-même équidistante des montants 21. Une seconde ligne 8 perpendiculaire à la ligne verticale médiane 7 est également tracée sur la latte 3, de préférence centrée sur la hauteur de la latte 3 et sécante de la ligne 7. Lesdites deux séries de graduations 6a, 6b sont reportées sur cette seconde ligne 8, laquelle constitue un moyen de repérage et de positionnement de la ligne bi-pupillaire d'un patient. La ligne 7 quant à elle forme un moyen de repérage et de positionnement de l'axe sagittal médian d'un patient. Les graduations quant à elles servent à mesurer l'écartement des pupilles par rapport au dit axe sagittal médian et leur symétrie relative par rapport à cet axe marqué par la ligne 7.

Dans le prolongement de la ligne 7, la latte 3 comporte une échancrure nasale pour permettre un rapprochement du cadre au plus proche du visage du patient lors du repérage et des mesures des paramètres esthétiques particuliers dudit patient comme il sera développé par la suite.

Au dessus de la traverse 22 inférieure du cadre 2 le dispositif 1 comporte également des moyens de positionnement et de matérialisation de la ligne du sourire, ou ligne incisive du maxillaire supérieur. Ces moyens consistent plus particulièrement en un fil 9, de matière synthétique ou naturelle, fixé à ces extrémités de manière coulissante sur les montants 21 du cadre 2. Pour faciliter la mesure de la position verticale de cette ligne incisive, lesdits montants 21 comportent chacun des graduations 10a, 10b, dans lesquelles les extrémités du fil peuvent être bloquées après coulissement.

Afin d'obtenir un positionnement du cadre 2 parfaitement fixe lors des prises de mesures, le dispositif de l'invention comporte un moyen original de positionnement du cadre 2 relativement au visage des patients qui consiste en une fourchette de morsure 11 solidaire de la traverse 22 inférieure du cadre 2 et mobile sur et autour de celle-ci par l'intermédiaire d'une liaison articulée 12 apte à coulisser et pivoter sur ladite traverse 22.

Comme représenté sur les figures 2A et 2B la fourchette de morsure 11 comporte une tige 111 rectiligne permettant la fixation de la fourchette 11 sur la traverse inférieure 22 du cadre 2 par l'articulation 12 et un plateau 112 de morsure à une extrémité de ladite tige.

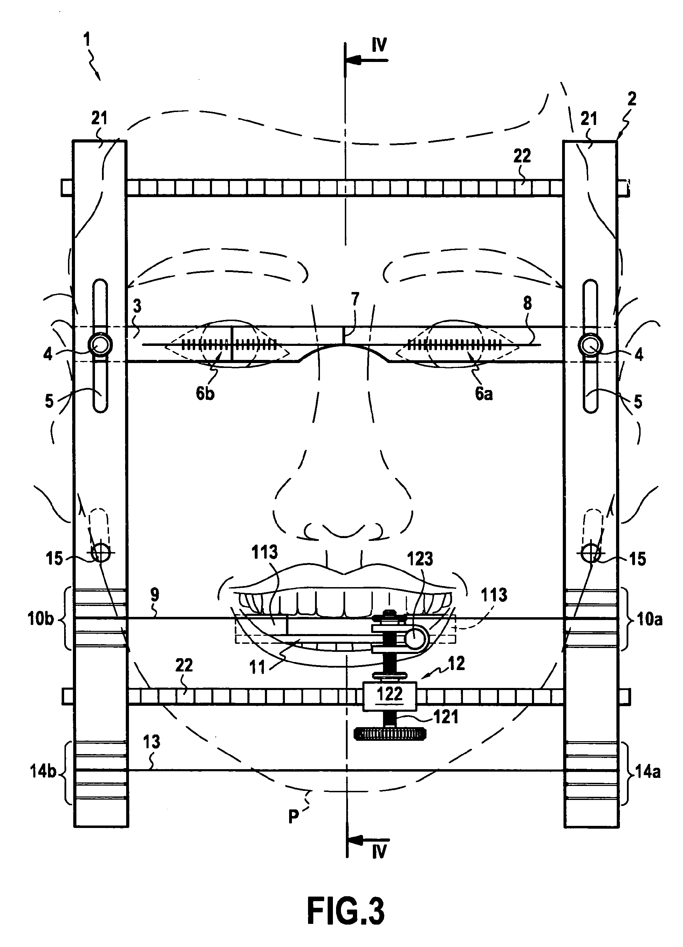

De préférence, la tige 111 est déportée dans le prolongement d'un bord du plateau 112. Ceci permet une meilleure visibilité des incisives des patients lors des mesures à l'aide du dispositif de l'invention par décalage latéral de l'articulation 12 par rapport aux incisives du patient dont le bord inférieur doit être visualisé et relevé pour matérialiser la ligne incisive et sa position comme il sera décrit ci-après à la figure 3.

Le plateau 112 de morsure est préférentiellement de relativement faible longueur comparativement à ceux des fourchettes habituellement employées dans le domaine dentaire. Il présente une forme sensiblement en U dont les deux branches portent en outre, conformément à une caractéristique spécifique de l'invention deux patins 113 de désocclusion, placés sur la face supérieure desdites branches. Ces patins 113 ont une forme sensiblement triangulaire rectangle, d'épaisseur plus faible à l'extrémité des branches du plateau. De préférence, l'épaisseur maximale desdits patins 113 est de l'ordre de 5 mm. Ces patins présentent avantageusement des moyens de repérage et/ou d'enregistrement de l'endroit exact où le patient mord. Ces moyens, qui peuvent être réalisés à l'aide d'éléments de moulage des dents postérieures au niveau des patins ou encore avec des moyens d'enregistrement, servent ensuite pour positionner de manière exacte le moulage en plâtre sur le cadre du dispositif selon l'invention.

La liaison articulée 12 comporte une vis de serrage 121 sur la tige de laquelle sont vissées deux pattes 122, 123 en U, la patte 122 étant en prise sur la traverse 22 et la patte 123 étant en prise sur la tige 111 de la fourchette de morsure 11. La vis 121 comporte à une extrémité une molette de serrage par l'intermédiaire de laquelle il est possible d'augmenter ou de diminuer simultanément le serrage des pattes 122, 123 sur la traverse 22 et sur la fourchette 11 respectivement et de bloquer leur position.

En complément du fil 9 et des graduations 10a, 10b de positionnement et de matérialisation de la ligne incisive supérieure, le dispositif 1 de l'invention comporte un second fil 13, également fixé sur les montants 21 du cadre 2 et situé sous la traverse inférieure 22 portant la fourchette 11. Ce second fil 13 permet de repérer, dans certains cas cliniques qui l'exigent, la ligne d'incisive du maxillaire inférieur. Des graduations 14a, 14b, identiques aux graduations 10a, 10b sont également formées sur les montants et permettent le réglage et la mesure du niveau de cette ligne incisive inférieures.

Enfin, le dispositif de l'invention comporte également des tiges ou bras latéraux 15 encastrés en une de leurs extrémités dans les montants 21 du cadre 2 et qui s'étendent, sur la figure 1, dans un plan perpendiculaire au plan du cadre 2. On constate que l'extension de cette tige 15 de manière perpendiculaire au plan du cadre n'est pas obligatoire. En particulier, pour pouvoir adapter le cadre quelle que soit la taille de la tête du patient et en conservant une taille minimale au cadre, il sera judicieux d'encastrer les tiges 15 de manière à ce que les plans les contenant fassent un angle différent de 90° avec le plan du cadre de part et d'autre du cadre vers l'extérieur de celui-ci. Avec ces tiges divergentes, le placement du visage du patient entre elles est facilité.

Ces tiges 15 ont pour fonction de repérer sur les profils des visages des patients un plan parallèle au plan anatomique de Camper PC, lequel passe par le porion et le point sous nasal. C'est pourquoi lesdites tiges forment un angle α compris entre 60° et 80° avec les montants 21, angle qui correspond aux variations anatomiques moyennes d'inclinaison du Plan de Camper PC.

Dans un mode de réalisation avantageux de l'invention, un ensemble de plusieurs emplacements sont prévus sur chacun des deux montants 21 du cadre pour pouvoir recevoir chacune des tiges 15. Ces emplacements sont avantageusement matérialisés par une pluralité d'orifices pratiqués dans chacun des montants 21, ces orifices étant tels que la tige étant placée dans un de ces orifices fait l'angle α compris entre 60° et 80° avec le montant 21 sur lequel elle est placée. La possibilité de changer la hauteur des tiges 15 permet un repérage plus aisé du plan de Camper en partant du principe que plus la tige est proche en hauteur du plan de Camper (ligne point sous-nasal/porion) observée sur le patient, plus l'alignement de la tige avec cette ligne fictive est aisée.

Le procédé complet d'utilisation du dispositif de l'invention 1 va maintenant être décrit en relation avec les figures 3 à 6B, dans le cadre d'un traitement de réparation ou de reconstruction maxillaire supérieure. Une des difficultés majeures de ces opérations réside dans la préparation de prothèses dentaires conformées pour respecter le parallélisme de la ligne incisive idéale et des gencives avec la ligne bi-pupillaire et le parallélisme de la ligne inter-incisives avec l'axe sagittal médian du visage. Le dispositif 1 de l'invention permet d'évaluer le positionnement exact de la ligne bi-pupillaire, de l'axe sagittal médian et de la ligne incisive sur le visage d'un patient.

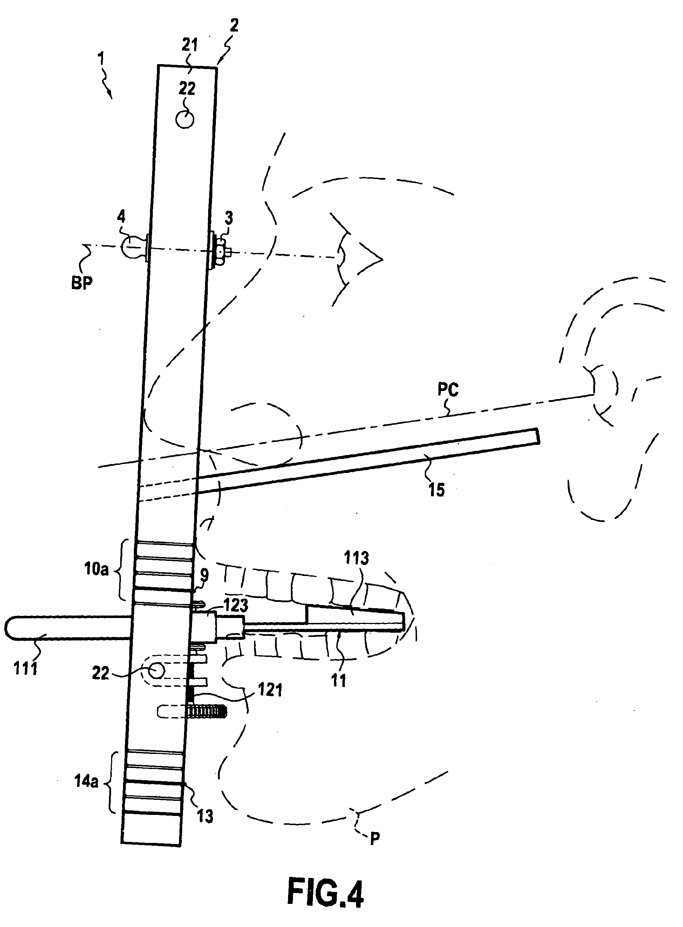

En référence aux figures 3 et 4 dans un premier temps, le dispositif 1 est mis en place en regard du visage d'un patient P par introduction de la fourchette de morsure dans la bouche du patient. L'articulation 12 de la fourchette 11 sur la traverse inférieure 22 du cadre est alors desserrée de manière à permettre une mobilité du cadre 2 et de la fourchette 11 suffisante à un bon ajustement de la fourchette en bouche. La fourchette 11 doit être mordue par le patient entre les molaires supérieures et inférieures.

La présence des patins 113 sur le plateau 112 de la fourchette provoque alors une désocclusion des maxillaires supérieur et inférieur. De préférence, on dépose sur les patins 113 avant mise en bouche de la fourchette 11 une fine couche de cire ou d'un autre matériau de moulage qui permettra de personnaliser les plans de morsure aux arcades dentaires du patient P et également de repérer la position exacte de morsure lors de la mise en place ultérieure du modèle moulé en plâtre. Dans le même temps la fourchette se trouve fermement maintenue en position dans la bouche du patient P. On remarque que la fourchette doit être mordue au niveau des dents postérieures pour assurer la stabilité du dispositif et la fiabilité des mesures de position par rapport au visage du patient.

On règle alors la position du cadre 2 par rapport au visage du patient par pivotement et glissement du cadre sur la traverse inférieure 22 au niveau de l'articulation 12 et glissement de ladite articulation sur la tige 111 de la fourchette 11 de manière à placer le cadre 2 au plus proche de la face du visage du patient P, et de telle façon que, d'une part la ligne verticale 7 tracée sur la latte 3, dite également le réglet, soit alignée avec l'axe sagittal médian S du visage du patient P et que, d'autre part les tiges latérales 15 sont parallèles au Plan de Camper PC, tel que représenté à la figure 4.

Dans l'exemple spécifique de la figure 4, le patient observé ne remplit pas les critères esthétiques reconnus concernant le parallélisme entre le plan de Camper et le plan d'occlusion. Ce sont des situations courantes mais qui peuvent précisément être résolues lors des reconstitutions de dentition par techniques prothétiques et ce, grâce au dispositif selon l'invention.

Une fois cette position trouvée, on serre la molette de la tige 121 de l'articulation 12 pour bloquer la position du cadre 2, qui reste ainsi en équilibre par la force appliquée par la mâchoire du patient P sur la fourchette 11.

On ajuste ensuite la position en hauteur de la latte 3 par rapport au visage à l'aide des boulons 4 dans les rainures 5 pour positionner et matérialiser la ligne bi-pupillaire. Cette visée s'effectue en utilisant la ligne 8 tracée sur la latte 3 et en alignant cette ligne avec les pupilles des yeux du patient P. Par construction, la latte 3 se déplace dans une direction parallèle aux montants du cadre en restant perpendiculaire à ceux-ci. Cet alignement obtenu, on serre les boulons 4 et on peut ensuite déterminer grâce aux graduations 6a et 6b l'écartement exact des pupilles par rapport à l'axe sagittal médian S matérialisé par la ligne 7 sur la latte 3 et, le cas échéant, mesurer les dissymétries entre les deux yeux du patient, ou, plus simplement de recentrer correctement le cadre.

La ligne bi-pupillaire fixée par la ligne 8, on repère ensuite la ligne du sourire ou ligne incisive du maxillaire supérieur grâce au fil 9. Pour ce repérage, la désocclusion générée par les patins 113 de la fourchette 11 joue un rôle important en ce qu'ils permettent une visualisation du bord inférieur des incisives non perturbée par l'articulation 12 de fixation du cadre 2 et de la fourchette 11. On peut ainsi exactement aligner le fil 9 et la ligne d'incisive du patient et relever, grâce aux graduations 10a, 10b sur les montants 21 du cadre la position exacte de cette ligne.

La ligne bi-pupillaire et la ligne incisive repérée sur le visage du patient, le praticien peut diagnostiquer d'éventuelles asymétries qu'il pourra progressivement corriger.

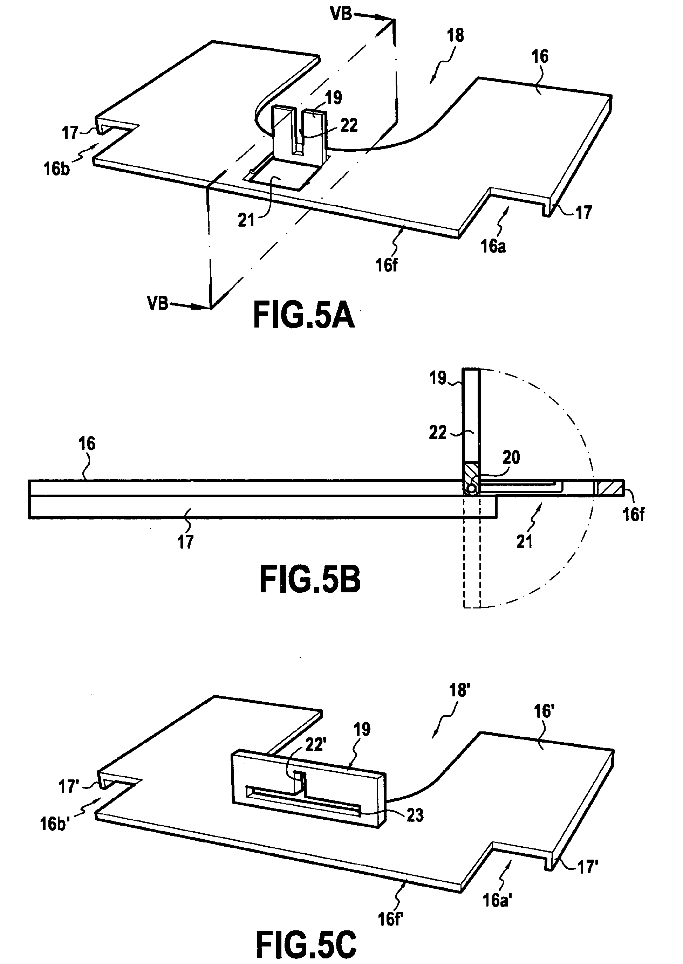

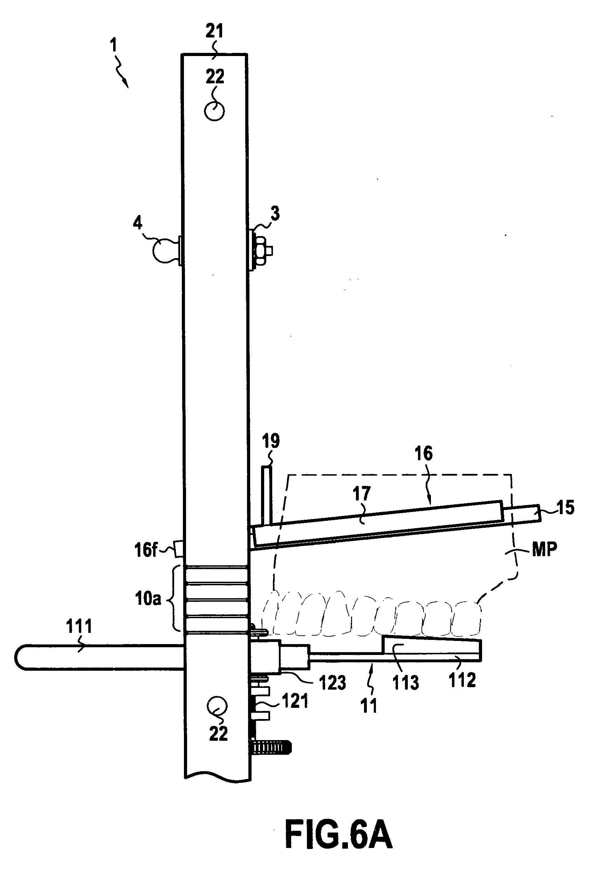

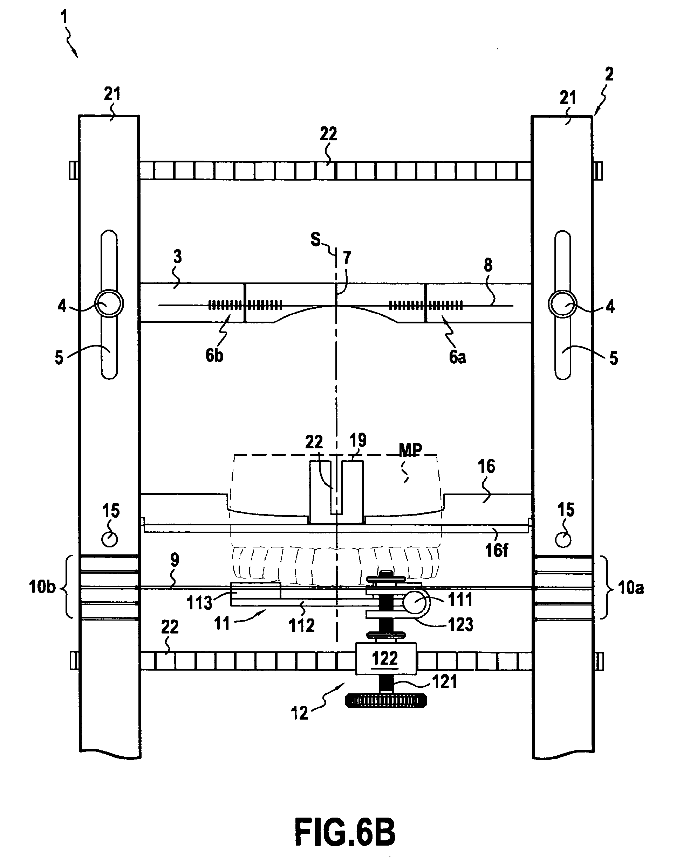

Le dispositif de l'invention comporte également un outil de transfert de repères esthétiques sur un moulage maxillaire de plâtre servant à la réalisation des prothèses dentaires afin que ces dernières procurent après leur mise en place une impression d'esthétique générale du visage respectée. Deux modes de réalisation de cet outil de transfert sont représentés aux figures 5A à 5C et leur utilisation est décrite aux figures 6A et 6B.

Dans les deux modes de réalisation présenté sur la figure 5, l'outil de transfert consiste en un plateau 16 ou respectivement 16' de forme générale rectangulaire de longueur sensiblement égale à l'écartement maximal entre les tiges 15 latérales du cadre et de largeur quelconque, comportant deux découpes frontales 16a, 16b (ou 16a', 16b') rectangulaires aux coins du plateau 16 ou 16'. De plus, le plateau comporte également sur ses deux bords latéraux 16c, 16d (ou 16c', 16d') des languettes 17 ou 17'.

Ces découpes 16a, 16b (ou 16a', 16b') et ces languettes 17 ou 17' sont adaptées pour permettre un glissement et stabilisation du plateau 16 ou 16' sur les tiges 15 du cadre 2 du dispositif et une insertion du bord frontal 16f ou 16f' entre les montants 21 jusqu'à venir en butée contre lesdits montants 21. La longueur des découpes 16a, 16b (ou 16a', 16b') est de préférence choisie telle que le bord frontal 16f ou 16f' du plateau dépasse entre les montants 21 lorsque le plateau est en butée contre les montants de 2 à 3 cm.

Le plateau 16 ou 16' comporte également une ouverture postérieure 18 ou 18' de forme adaptée pour permettre le positionnement dans cette ouverture 18 ou 18' d'un modèle maxillaire en plâtre comme représenté aux figures 6A et 6B. Cette ouverture 18 ou 18' est centrée dans la longueur du plateau 16 ou 16' et son bord avant, sensiblement circulaire sur les figures se trouve sensiblement dans le plan de la face arrière des montants 21 du cadre 2 du dispositif lorsque le plateau 16 ou 16' est en butée contre les montants 21.

Sur les figures 5A et 5B, le plateau 16 comporte une languette pivotante 19 située entre l'ouverture 18 et le bord frontal du plateau 16f. Cette languette 19 est montée pivotante et coulissante autour de deux ergots 20 logés dans une rainure ou ouverture pratiquée dans les bordures d'une découpe rectangulaire 21 formée dans le plateau 16 et comporte une rainure centrale 22 située dans le plan transversal médian VB du plateau, lequel plan VB est également médian à l'ouverture 18.

Quand elle est perpendiculaire au plateau 16, la languette 19 sert à transférer sur des modèles maxillaires en plâtre une ligne représentative de l'axe sagittal médian S représentée par la ligne 7 sur la latte 3 du dispositif. Le plateau lui-même sert, une fois la languette rabattue, pour reporter une ligne parallèle à la ligne bi-pupillaire BP, matérialisées sur le dispositif 1 de l'invention par la ligne 8 sur la latte transparente 3 et le fil 9.

Pour effectuer ce transfert, on procède comme représenté aux figures 6A et 6B et comme explicité dans la suite.

Sur la figure 5C, le plateau 16' comporte une languette fixe 19' située entre l'ouverture 18' et le bord frontal du plateau 16f'. Cette languette 19' est montée de manière fixe, par exemple à l'aide de deux vis traversant le plateau 16'. Elle comporte deux rainures 22' et 23 dans la région centrale de la languette 19' située dans le plan transversal médian VB du plateau, lequel plan VB est également médian à l'ouverture 18'.

La languette 19' sert ici, à elle seule, à transférer sur des modèles maxillaires en plâtre une ligne représentative de l'axe sagittal médian S représentée par la ligne 7 sur la latte 3 du dispositif ainsi qu'une ligne parallèle à la ligne bi-pupillaire BP, matérialisées sur le dispositif 1 de l'invention par la ligne 8 sur la latte transparente 3 et le fil 9. Le plateau sert à reporter une ligne parallèle au plan de Camper sur les cotés du modèle.

Dans la suite est explicitée la manière de reporter des repères pris sur le patient sur un modèle moulé en plâtre avec le plateau des figures 5A et 5B. On place le plateau 16 sur les tiges 15 du cadre 2 et on le bloque en butée contre les montants 21 du cadre 2 au niveau des découpes d'angle 16a, 16b.

On positionne ensuite un modèle maxillaire en plâtre MP sur les patins 113 de la fourchette 11 du dispositif 1 grâce à l'indentement des secteurs postérieur sur la cire d'enregistrement qui recouvre les patins 113 de ladite fourchette, dont on n'aura pas modifié les réglages après prise de repères sur le visage du patient. Ainsi le modèle MP se trouve-t-il exactement dans une position identique à celle du maxillaire supérieur du patient lors de la prise de repères (figures 3 et 4). Le modèle MP se situe alors dans l'ouverture 18 du plateau 16 reposant sur les tiges latérales 15 du dispositif.

Ensuite, à l'aide d'un crayon, on reporte sur le modèle MP une ligne horizontale en faisant coulisser le crayon sur le plan du plateau 16, puis après avoir relevé la languette 19 on reporte une ligne verticale en guidant le crayon dans la rainure ou ouverture ou fenêtre centrale 22 de la languette pivotante 19. En faisant pivoter ladite languette 19 vers le bas on peut ensuite prolonger la ligne verticale par le dessous du plateau 16. Ces deux lignes permettront ensuite au prothésiste d'avoir un repère exact de la parallèle à la ligne bi-pupillaire, du plan de Camper et de l'axe sagittal médian du patient sur le modèle en plâtre MP.

Une fois ces lignes horizontales et verticales tracées sur le modèle maxillaire en plâtre MP par le praticien, ce dernier bénéficie d'un repère orthogonal dont l'axe horizontal est parallèle à la ligne bi-pupillaire et l'axe vertical est perpendiculaire à celle-ci, ce qui permet de réaliser une prothèse dentaire qui produira un rendu esthétique parfait après mise en place, permettant au patient de retrouver une esthétique du sourire normale.

En outre, le plateau 16 étant placé sur les tiges parallèles au plan de Camper, il offre la possibilité de tracer, sur les cotés du modèle en plâtre, une ligne également parallèle au plan de Camper ainsi que représenté sur la figure 6A. Dans un cas esthétique idéal, cette ligne, parallèle au plan de Camper, est, selon les critères morphologiques idéaux, parallèle à la ligne d'occlusion idéale. Cela est utilisé pour la reconstruction le cas échéant. Sur la figure 6A, la mâchoire montrée ne nécessite pas de reconstruction mais il peut être noté que le plan d'occlusion n'est pas parallèle au plan de Camper. Le patient correspondant ne présente pas des paramètres morphologiques idéaux.

## Revendications

1. Dispositif (1) de repérage et de mesure de paramètres anatomiques faciaux d'un visage humain, **caractérisé en ce qu'**il comporte:
- un cadre (2) destiné à être positionné face à un dit visage et comportant au moins deux montants (21), et
- une fourchette de morsure (11) comprenant une tige de fixation (111) montée sur une traverse (22) dudit cadre, ladite fourchette (11) étant apte à pivoter autour de ladite traverse (22) et autour de l'axe de la tige de fixation (111) et étant destinée à être insérée dans la bouche et mordue par un patient, et
- des moyens (9, 10a, 10b) de matérialisation et de positionnement de la ligne incisive, solidaires du cadre (2) et aptes à coulisser pour être alignés avec la ligne incisive d'un patient, et
- des moyens (3, 4, 5, 6a, 6b, 8) de matérialisation et de positionnement de la ligne bi-pupillaire solidaires du cadre (2), perpendiculaires aux montants verticaux (21), et aptes à coulisser pour être alignés avec la ligne bi-pupillaire d'un patient, et
- des moyens (15) d'alignement du plan de Camper sur au moins un profil dudit visage, solidaires dudit cadre (2) comportant au moins une tige (15) rectiligne fixée sur au moins un des montants dudit cadre (2) et destinée à être placée parallèlement au plan de Camper du patient par pivotement du cadre (2) autour de la traverse (22) sur laquelle est montée la fourchette (11),
- un outil de transfert (16) apte à être positionné sur les moyens de positionnement du plan de Camper (15), cet outil de transfert permettant le transfert, sur un modèle maxillaire en plâtre (MP) des patients traités, d'une ligne parallèle à la ligne bi-pupillaire, qui est parallèle à la ligne incisive idéale, et, l'outil de transfert étant positionné sur les moyens de positionnement du plan de Camper, cet outil de transfert également permettant le transfert d'une ligne tridimensionnelle sur l'avant et les cotés du modèle, cette ligne appartenant à un plan parallèle au plan de Camper et étant, sur l'avant du modèle, parallèle à la ligne bi-pupillaire.

2. Dispositif selon la revendication 1, **caractérisé en ce que** les moyens (9, 10a, 10b) de matérialisation et de positionnement de la ligne incisive, et les moyens (3, 4, 5, 6a, 6b, 8) de matérialisation et de positionnement de la ligne bi-pupillaire comportent des moyens de blocage (10a, 10b, 4, 5) sur les montants (21) dudit cadre (2) dans une position de repérage souhaitée.

3. Dispositif de repérage et de mesure selon l'une des revendications 1 et 2, **caractérisé en ce que** ladite fourchette de morsure (11) est fixée sur la traverse (22) inférieure dudit cadre (2) par l'intermédiaire d'une pièce de liaison articulée (12) comprenant des moyens de serrage (121, 122, 123) variable permettant au moins des déplacements et blocages de ladite fourchette (11) en translation et en rotation sur ladite traverse (22) et autour de l'axe de sa tige de fixation (111).

4. Dispositif de repérage et de mesure selon l'une des revendications 1 à 3, **caractérisé en ce que** ladite fourchette de morsure (11) comprend des moyens d'enregistrement capable de garder trace de la position des surfaces dentaires du patient lorsqu'il mord la fourchette (11).

5. Dispositif de repérage et de mesure selon l'une des revendications 1 à 4, **caractérisé en ce que** ladite fourchette de morsure (11) comporte des patins (113) de désocclusion des mâchoires lorsque ladite fourchette (11) est mordue par un dit patient.

6. Dispositif de repérage et de mesure selon l'une des revendications 1 à 5, **caractérisé en ce que** lesdits moyens d'alignement du plan de Camper (15) forment un angle α compris entre 60° et 80° par rapport à au moins un des montants (21) dudit cadre (2).

7. Dispositif de repérage et de mesure selon l'une des revendications 1 à 6, **caractérisé en ce que** lesdits moyens de matérialisation et de positionnement de la ligne incisive et de la ligne bi-pupillaire (3, 4, 5, 6a, 6b, 8) comportent respectivement un fil (9) fixé de façon coulissante aux montants (21) du cadre et un réglet (3) transparent monté dans au moins une glissière (5) sur au moins un des montants (21) du cadre.

8. Dispositif de repérage et de mesure selon l'une des revendications 1 à 7, **caractérisé en ce qu'**il comporte également un moyen de positionnement (7) de l'axe sagittal médian (S) du visage.

9. Dispositif de repérage et de mesure selon la revendication 8, **caractérisé en ce que** ledit moyen (7) de positionnement de l'axe sagittal médian (S) du visage est solidaire desdits moyens (3, 4, 5, 6a, 6b, 8) de matérialisation et de positionnement de la ligne bi-pupillaire.

10. Dispositif de repérage et de mesure selon l'une des revendications 8 ou 9, **caractérisé en ce que** lesdits moyens de matérialisation et de positionnement de la ligne bi-pupillaire comportent des graduations (6a, 6b) permettant le centrage dudit moyen de positionnement de l'axe sagittal médian (S) par rapport aux pupilles dudit patient.

11. Dispositif de repérage et de mesure selon l'une des revendications précédentes1, **caractérisé en ce que** ledit outil de transfert comporte un plateau (16) muni d'une ouverture postérieure (18) permettant l'ajustement du plateau autour d'un dit modèle maxillaire de plâtre (MP) placé sur la fourchette (11) du dispositif ainsi que de découpes frontales (16a, 16b) permettant le placement en butée dudit plateau contre les montants (21) du cadre et, perpendiculairement aux montants (21) du cadre, en appui sur lesdits moyens d'alignement du plan de Camper (15).

12. Dispositif de repérage et de mesure selon la revendication 11, **caractérisé en ce que** ledit plateau (16) comporte une languette (19), pivotante ou fixe, munie d'au moins une rainure centrale (20) permettant le tracé sur un dit modèle maxillaire de plâtre (MP) d'une ligne représentative de l'axe sagittal médian (S) du visage du patient.

13. Dispositif de repérage et de mesure selon la revendication 12, **caractérisé en ce que** la languette est munie de rainures en T ou en croix permettant le tracé sur un dit modèle maxillaire de plâtre (MP) d'une ligne représentative de l'axe sagittal médian (S) du visage du patient et d'une ligne parallèle à la ligne bi-pupillaire.

## Claims

1. A locating and measuring device (1) of the anatomical facial parameters of a human face, **characterised in that** it includes:
- a frame (2) intended to be positioned in front of a said face and including at least two uprights (21), and
- a bite yoke (11) that includes a securing rod (111) mounted on a cross-member (22) of the said frame, the said yoke (11) being designed to pivot around the said cross-member (22) and around the axis of the securing rod (111) and being intended to be inserted into the mouth and bitten onto by a patient, and
- means (9,10a,10b) for materialisation and positioning of the incisive line, attached to the frame (2) and designed to slide so as to be aligned with the incisive line of a patient, and
- means (3,4,5,6a,6b,8) for materialisation and positioning of the bi-pupillary line attached to the frame (2), perpendicular to the vertical uprights (21), and designed to slide so as to be aligned with the bi-pupillary line of a patient, and
- means (15) for alignment of the Camper's plane on at least one profile of the said face, attached to the said frame (2), and including at least one rectilinear rod (15) fixed onto at least one of the uprights of the said frame (2) and intended to be placed parallel to the Camper's plane of the patient by pivoting of the frame (2) around the cross-member (22) on which the yoke (11) is mounted,
- a transfer tool (16) designed to be positioned on the positioning means of the Camper's plane, this transfer tool allowing the transfer, onto a plaster maxillary model (MP) of the patients treated, of a line parallel to the bi-pupillary line, which is parallel to the ideal incisive line and, the transfer tool being positioned on the positioning means of the Camper's plane, this transfer tool further allowing the transfer of a three-dimensional line onto the front and sides of the model, this line belonging to a plane parallel to the Camper's plane and being parallel to the bi-pupillary line at the front of the model.

2. A device according to claim 1, **characterised in that** the means (9,10a,10b) for materialisation and positioning of the incisive line, and the means (3,4,5,6a,6b,8) for materialisation and positioning of the bi-pupillary line include locking means (10a,10b,4,5) on the uprights (21) of the said frame (2) in a desired location position.

3. A locating and measuring device according to one of claims 1 and 2, **characterised in that** the said bite yoke (11) is fixed onto the lower cross-member (22) of the said frame (2) by means of an articulated connecting piece (12) that includes variable clamping means (121,122,123) that at least allow movement and locking of the said yoke (11) in translation and in rotation on the said cross-member (22) and around the axis of its securing rod (111).

4. A locating and measuring device according to one of claims 1 to 3, **characterised in that** the said bite yoke (11) includes recording means that are capable of keeping a trace of the position of the dental surfaces of the patient when he bites onto the yoke (11).

5. A locating and measuring device according to one of claims 1 to 4, **characterised in that** the said bite yoke (11) includes pads (113) for de-occlusion of the jaws when the said yoke (11) is bitten onto by a said patient.

6. A locating and measuring device according to one of claims 1 to 5, **characterised in that** the said means for alignment of the Camper's plane form a angle of between 60° and 80° in relation to at least one of the uprights of the said frame.

7. A locating and measuring device according to one of claims 1 to 6, **characterised in that** the said means (3,4,5,6a,6b,8) for materialisation and positioning of the incisive line and of the bi-pupillary line respectively include a wire (9) mounted so that it slides on the uprights (21) of the frame, and a transparent ruler (3) mounted in at least one slide (5) on at least one of the uprights (21) of the frame.

8. A locating and measuring device according to one of claims 1 to 7, **characterised in that** it also includes a positioning means (7) of the median sagittal axis (S) of the face.

9. A locating and measuring device according to claim 8, **characterised in that** the said positioning means (7) of the median sagittal axis (S) of the face is attached to the said means (3,4,5,6a,6b,8) for materialisation and positioning of the bi-pupillary line.

10. A locating and measuring device according to one of claims 8 and 9, **characterised in that** the said means for materialisation and positioning of the bi-pupillary line include graduations (6a,6b) used to centre the said positioning means of the median sagittal axis (S) in relation to the pupils of the said patient.

11. A locating and measuring device according to one preceding claims, **characterised in that** the said transfer tool includes a plate (16) fitted with a rear opening (18) used for adjustment of the plate around a said plaster maxillary model (MP) placed on the yoke (11) of the device, as well as frontal cut-outs (16a,16b) used for placement of the said plate up against the uprights (21) of the frame and, perpendicularly to the uprights (21) of the frame, bearing onto the said means (15) for alignment of the Camper's plane.

12. A locating and measuring device according to claim 11, **characterised in that** the said plate (16) includes a pivoting or fixed languet or tab (19) equipped with at least one central groove (22) or opening used to trace, onto a said plaster maxillary model (MP), a line representing the median sagittal axis (S) of the face of the patient.

13. A locating and measuring device according to claim 12, **characterised in that** the languet or tab (19) is fitted with grooves in the T-shape or a cross, used to trace onto a said plaster maxillary model (MP) a line representing the median sagittal axis (S) of the face of the patient, and a line parallel to the bi-pupillary line.

## Patentansprüche

1. Vorrichtung (1) zur Erfassung und Messung anatomischer Gesichtsparameter eines menschlichen Gesichts, **dadurch gekennzeichnet, daß** sie umfaßt:
- einen Rahmen (2), der dazu bestimmt ist, gegenüber einem Gesicht positioniert zu werden, und der wenigstens zwei Ständer (21) umfaßt, und
- eine Bißgabel (11), die eine an einer Traverse (22) des Rahmens angebrachte Fixierstange (111) umfaßt, wobei die Gabel (11) geeignet ist, um die Traverse (22) und um die Achse der Fixierstange (111) zu verschwenken, und dazu bestimmt ist, in den Mund eingeführt und von einem Patienten beißend erfaßt zu werden, sowie
- Mittel (9, 10a, 10b) zum Materialisieren und Positionieren der Inzisal-Linie, die mit dem Rahmen (2) fest verbunden und geeignet sind, verschoben zu werden, um mit der Inzisal-Linie eines Patienten ausgerichtet zu werden, und
- Mittel (3, 4, 5, 6a, 6b, 8) zum Materialisieren und Positionieren der Bipupillarlinie, die mit dem Rahmen (2) fest verbunden sind, zu den Vertikalstützen (21) senkrecht verlaufen und geeignet sind, verschoben zu werden, um mit der Bipupillarlinie eines Patienten ausgerichtet zu werden, und
- Mittel (15) zum Ausrichten der Camperschen Ebene auf wenigstens ein Profil des Gesichts, die mit dem Rahmen (2) fest verbunden sind, umfassend wenigstens eine geradlinige Stange (15), die an wenigstens einem der Ständer des Rahmens (2) befestigt und dazu bestimmt ist, durch Verschwenken des Rahmens (2) um die Traverse (22), an der die Gabel (11) angebracht ist, parallel zur Camperschen Ebene des Patienten plaziert zu werden,
- ein Transferwerkzeug (16), das geeignet ist, an den Mitteln zum Positionieren der Camperschen Ebene (15) positioniert zu werden, wobei dieses Transferwerkzeug das Übertragen einer Linie, welche zu der Bipupillarlinie parallel verläuft, die zu der idealen Inzisal-Linie parallel ist, auf ein Gips-Kiefermodell (MP) der behandelten Patienten ermöglicht, und wobei das Transferwerkzeug an den Mitteln zum Positionieren der Camperschen Ebene positioniert ist, wobei dieses Transferwerkzeug auch das Übertragen einer dreidimensionalen Linie auf den vorderen Teil und die Seiten des Modells ermöglicht, wobei diese Linie zu einer zu der Camperschen Ebene parallelen Ebene gehört und am vorderen Teil des Modells parallel zur Bipupillarlinie verläuft.

2. Vorrichtung nach Anspruch 1, **dadurch gekennzeichnet, daß** die Mittel (9, 10a, 10b) zum Materialisieren und Positionieren der Inzisal-Linie sowie die Mittel (3, 4, 5, 6a, 6b, 8) zum Materialisieren und Positionieren der Bipupillarlinie Mittel zum Festlegen (10a, 10b, 4, 5) an den Ständern (21) des Rahmens (2) in einer gewünschten Erfassungsposition umfassen.

3. Erfassungs- und Meßvorrichtung nach einem der Ansprüche 1 und 2, **dadurch gekennzeichnet, daß** die Bißgabel (11) an der unteren Traverse (22) des Rahmens (2) mittels eines Gelenkverbindungsteils (12) befestigt ist, das Mittel (121, 122, 123) zum variablen Festklemmen umfaßt, die wenigstens Verschiebe- und Drehbewegungen sowie -blockierungen der Gabel (11) an der Traverse (22) und um die Achse ihrer Fixierstange (111) ermöglichen.

4. Erfassungs- und Meßvorrichtung nach einem der Ansprüche 1 bis 3, **dadurch gekennzeichnet, daß** die Bißgabel (11) Registriermittel umfaßt, die geeignet sind, die Position der Zahnflächen des Patienten aufzuzeichnen, wenn dieser auf die Gabel (11) beiβt.

5. Erfassungs- und Meßvorrichtung nach einem der Ansprüche 1 bis 4, **dadurch gekennzeichnet, daß** die Bißgabel (11) Klötze (113) zur Bißsperrung der Kiefer, wenn ein Patient auf die Gabel (11) beißt, umfaßt.

6. Erfassungs- und Meßvorrichtung nach einem der Ansprüche 1 bis 5, **dadurch gekennzeichnet, daß** die Mittel zum Ausrichten der Camperschen Ebene (15) einen Winkel α zwischen 60° und 80° gegenüber wenigstens einem der Ständer (21) des Rahmens (2) bilden.

7. Erfassungs- und Meßvorrichtung nach einem der Ansprüche 1 bis 6, **dadurch gekennzeichnet, daß** die Mittel zum Materialisieren und Positionieren der Inzisal-Linie und der Bipupillarlinie (3, 4, 5, 6a, 6b, 8) einen Draht (9), der an den Ständern (21) des Rahmens verschieblich befestigt ist, bzw. ein transparentes Maßband (3), das in wenigstens einer Gleitschiene (5) an wenigstens einem der Ständer (21) des Rahmens angebracht ist, umfassen.

8. Erfassungs- und Meßvorrichtung nach einem der Ansprüche 1 bis 7, **dadurch gekennzeichnet, daß** sie auch ein Mittel zum Positionieren (7) der mittleren Sagittalachse (S) des Gesichts umfaßt.

9. Erfassungs- und Meßvorrichtung nach Anspruch 8, **dadurch gekennzeichnet, daß** das Mittel (7) zum Positionieren der mittleren Sagittalachse (S) des Gesichts mit den Mitteln (3, 4, 5, 6a, 6b, 8) zum Materialisieren und Positionieren der Bipupillarlinie fest verbunden ist.

10. Erfassungs- und Meßvorrichtung nach einem der Ansprüche 8 oder 9, **dadurch gekennzeichnet, daß** die Mittel zum Materialisieren und Positionieren der Bipupillarlinie Skalen (6a, 6b) umfassen, die das Zentrieren des Mittels zum Positionieren der mittleren Sagittalachse (S) gegenüber den Pupillen des Patienten ermöglichen.

11. Erfassungs- und Meßvorrichtung nach einem der vorhergehenden Ansprüche, **dadurch gekennzeichnet, daß** das Transferwerkzeug eine Platte (16) umfaßt, die mit einer hinteren Öffnung (18), welche das Justieren der Platte um ein an der Gabel (11) der Vorrichtung angeordnetes Gips-Kiefermodell (MP) ermöglicht, sowie mit stirnseitigen Ausschnitten (16a, 16b) versehen ist, die ermöglichen, die Platte in Anschlag an den Ständern (21) des Rahmens sowie, senkrecht zu den Ständern (21) des Rahmens, in Anlage an den Mitteln zum Ausrichten der Camperschen Ebene (15) anzuordnen.

12. Erfassungs- und Meßvorrichtung nach Anspruch 11, **dadurch gekennzeichnet, daß** die Platte (16) eine verschwenkbare oder feste Lasche (19) umfaßt, die mit wenigstens einer mittleren Nut (22) versehen ist, welche das Anzeichnen einer die mittlere Sagittalachse (S) des Gesichts des Patienten darstellenden Linie an einem Gips-Kiefermodell (MP) ermöglicht.

13. Erfassungs- und Meßvorrichtung nach Anspruch 12, **dadurch gekennzeichnet, daß** die Lasche mit T-förmigen oder kreuzförmigen Nuten versehen ist, welche das Anzeichnen einer die mittlere Sagittalachse (S) des Gesichts des Patienten darstellenden Linie und einer zu der Bipupillarlinie parallelen Linie an einem Gips-Kiefermodell (MP) ermöglichen.
